(19) Europäisches Patentamt / European Patent Office / Office européen des brevets

(11) **EP 2 550 872 B1**

(12) **EUROPÄISCHE PATENTSCHRIFT**

(45) Veröffentlichungstag und Bekanntmachung des Hinweises auf die Patenterteilung:
**06.09.2017 Patentblatt 2017/36**

(51) Int Cl.:
*A23L 2/02* (2006.01)     *A23L 2/52* (2006.01)
*A23L 33/105* (2016.01)     *A23L 2/04* (2006.01)

(21) Anmeldenummer: **11176053.4**

(22) Anmeldetag: **29.07.2011**

(54) **VERFAHREN ZUM ERZEUGEN EINER ZUSAMMENSETZUNG UMFASSEND ANTHOCYANE SOWIE ENTSPRECHENDE ZUSAMMENSETZUNGEN**

METHOD FOR PRODUCING A COMPOUND COMPRISING ANTHOCYANE AND CORRESPONDING COMPOUNDS

PROCÉDÉ DE PRODUCTION D'UNE COMPOSITION COMPRENANT DE L'ANTHOCYANE ET COMPOSITIONS CORRESPONDANTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.01.2013 Patentblatt 2013/05**

(73) Patentinhaber: **Symrise AG**
**37603 Holzminden (DE)**

(72) Erfinder:
• **Kaßing, Markus**
**37671 Höxter (DE)**
• **Stürtz, Dr., Melanie**
**34385 Bad Karlshafen (DE)**
• **Erfurt, Harry**
**37170 Uslar-Schönhagen (DE)**
• **Niemeyer, Hans-Jürgen**
**37603 Holzminden (DE)**
• **Repenn, Florian**
**21039 hamburg (DE)**
• **Roloff, Michael**
**34399 Oberweser (DE)**

(74) Vertreter: **Fabry, Bernd**
**IP2 Patentanwalts GmbH**
**Schlossstrasse 523**
**41238 Mönchengladbach (DE)**

(56) Entgegenhaltungen:
**EP-A1- 1 602 652     US-A1- 2007 269 536**

• **KATARINA AA AVIKIN ET AL: "Phenolic Content and Radical Scavenging Capacity of Berries and Related Jams from Certificated Area in Serbia", PLANT FOODS FOR HUMAN NUTRITION, KLUWER ACADEMIC PUBLISHERS, DO, Bd. 64, Nr. 3, 26. Mai 2009 (2009-05-26), Seiten 212-217, XP019733279, ISSN: 1573-9104, DOI: 10.1007/S11130-009-0123-2**
• **ROUANET J M ET AL: "Berry juices, teas, antioxidants and the prevention of atherosclerosis in hamsters", FOOD CHEMISTRY, ELSEVIER LTD, NL, Bd. 118, Nr. 2, 15. Januar 2010 (2010-01-15), Seiten 266-271, XP026497666, ISSN: 0308-8146, DOI: 10.1016/J.FOODCHEM.2009.04.116 [gefunden am 2009-05-07]**

## Beschreibung

Die vorliegende Erfindung betrifft ein Verfahren zum Erzeugen einer Zusammensetzung umfassend die Anthocyane Delphinidin, Cyanidin, Petunidin, Peonidin und Malvidin in glykosidisch gebundener Form sowie eine Zusammensetzung umfassend diese glykosidisch gebundenen Anthocyane.

Zusammensetzungen umfassend die besagten Anthocyane Delphinidin, Cyanidin, Petunidin, Peonidin und Malvidin in glykosidisch gebundener Form sowie Verfahren zu deren Herstellung sind bereits bekannt. Die besagten glykosidisch gebundenen Anthocyane sind nämlich Bestandteile der Früchte von *Vaccinium myrtillus* L. (bilberry fruit). Die European Pharmacopoeia umfasst eine Monographie betreffend "Fresh bilberry fruit dry extract, refinded and standardised", aus der sich unter anderem ergibt, dass sämtliche der besagten Anthocyane in glykosidisch gebundener Form Bestandteile der Früchte von *Vaccinium myrtillus* L. sind.

Die besagten Anthocyane umfassen einen Anthocyanidin-Grundkörper, der an seiner Hydroxylgruppe am Kohlenstoffatom 3 über eine O-glykosidische Bindung an Galactose, Glucose oder Arabinose gebunden ist. Es resultieren somit insgesamt 15 verschiedene Anthocyane. Der Begriff "glykosidisch gebundenes Anthocyan" bezeichnet das 3-Glucosid, das 3-Arabinosid und das 3-Galactosid des betreffenden Anthocyanidin-Grundkörpers. Die "Gesamtmasse eines glykosidisch gebundenen Anthocyans" in einer Zusammensetzung entspricht der Gesamtmasse der drei jeweiligen Glykoside in der Zusammensetzung, also der Summe der Massen des jeweiligen 3-Glucosids, 3-Arabinosids und 3-Galactosids. Beispielsweise ist somit die Gesamtmasse des glykosidisch gebundenen Anthocyans Delphinidin in einer Zusammensetzung die Summe der Massen von Delphinidin-3-glucosid, Delphinidin-3-arabinosid und Delphinidin-3-galactosid in der Zusammensetzung.

Im nachfolgenden Text werden im Einklang mit dem fachmännischen Gebrauch der Begriffe die glykosidisch gebundenen Anthocyane in manchen Fällen auch einfach als "Anthocyane" bezeichnet. Im Unterschied dazu bezeichnet der Begriff "Anthocyanidine" die entsprechenden Aglykone. Dem deutschsprachigen Begriff "Anthocyane" entspricht der englischsprachige Begriff "anthocyanins", dem deutschsprachigen Begriff "Anthocyanidine" entspricht der englischsprachige Begriff "anthocyanidins".

Es gibt bereits eine Reihe von Handelsprodukten auf Basis von *Vaccinium myrtillus* L. (Heidelbeere, auch Blaubeere, Schwarzbeere genannt; in der englischen Sprache: Bilberry), welche die oben genannten Anthocyane umfassen.

Es sind auch bereits getrocknete Zusammensetzungen im Handel, die als "bilberry extract" bezeichnet werden und einen Anteil von ≥ 25 Gew.-% an den besagten glykosidisch gebundenen Anthocyanen umfassen.

Derartige Zusammensetzungen sind ebenso wie andere Zusammensetzungen, welche die oben genannten Anthocyane umfassen, zur Förderung der Darmgesundheit, zur Förderung der Gesundheit des Herz-Kreislaufsystems, als anti-inflammatorische Zusammensetzungen, zur Immunstärkung und zur Förderung der Augengesundheit beim Menschen ebenso wie beim Tier geeignet.

Wenngleich wie vorstehend ausgeführt bereits eine Vielzahl von Produkten auf Basis von Früchten von *Vaccinium myrtillus* L. bekannt sind, welche die oben genannten Anthocyane umfassen, insbesondere auch getrocknete Zusammensetzungen, welche einen Massenanteil von mehr als 25 Gew.-% der besagten Anthocyane umfassen, ist über schonende Verfahren zu ihrer Herstellung nicht viel mehr zu erfahren, als dass diese aus frischen Früchten von insbesondere *Vaccinium myrtillus* L. erhalten werden, die geerntet werden, wenn sie reif sind, d. h. beispielsweise zwischen Juli und September. Es ist bislang nur den Herstellern der im Handel erhältlichen getrockneten Zusammensetzungen mit einem Anteil an Anthocyanen ≥ 25 Gew.-% bekannt, wie ausgehend von diesen frischen Früchten das jeweilige Handelsprodukt erhalten wird. Damit die oben genannten Anthocyane in einer Zusammensetzung auf Basis von *Vaccinium myrtillus* L. zumindest in etwa die gleiche Funktion ausüben können, welche sie beim Verzehr von frischen Früchten bewirken, streben die Hersteller entsprechender Zusammensetzungen danach, ihr Massenverhältnis zumindest in etwa so zu bewahren, wie es in den Früchten von *Vaccinium myrtillus* L. vorliegt, dabei aber den Massenanteil der besagten Anthocyane im Vergleich zu dem Massenanteil der Anthocyane in den Früchten von *Vaccinium myrtillus* L. zu erhöhen. Es hat sich insoweit als sinnvoll erwiesen, das Verhältnis der Gesamtmassen der glykosidisch gebundenen Anthocyane Delphinidin, Cyanidin, Petunidin, Peonidin und Malvidin in einer entsprechenden Zusammensetzung so einzustellen, dass es im Bereich (3 - 8): (1,8 - 6): (0,5 - 5): 1: (0,5 - 5,5) liegt. Eine Reihe von Handelsprodukten, bei denen es sich um getrocknete Zusammensetzungen auf Basis von Früchten von *Vaccinium myrtillus* L. handelt, liegen im angegebenen Massenverhältnis-Bereich. Nicht bekannt ist jedoch, wie es den jeweiligen Herstellern im Einzelfall gelingt, ausgehend von den reifen Früchten von *Vaccinium myrtillus* L. zu einer getrockneten Zusammensetzung zu gelangen, welche die Anthocyane (noch immer) in einem entsprechenden Massenverhältnis umfasst und dabei den Massenanteil der besagten Anthocyane im Vergleich zu dem Massenanteil der Anthocyane in den Früchten von *Vaccinium myrtillus* L. zu erhöhen. Die bekannten Handelsprodukte umfassen zudem größere Mengen an Zuckern, was als nachteilig empfunden wird.

In US 2007/269536 A1 werden zwei Verfahren zur Herstellung einer Zusammensetzung, die Anthocyane umfassen, offenbart. Ausgangsmaterial in beiden Verfahren ist ein Extrakt aus Pflanzenmaterial, wobei alle Teile der Pflanzen dafür verwendet werden können. Als mögliche Fruchtquelle werden u.a. Heidelbeeren und Brombeeren verwendet. Im ersten Verfahren wird das Pflanzenmaterial mit einem Extraktionsmittel, welches eine Mischung aus Ethanol/Wasser oder Methanol/Wasser sein kann behandelt. Dem Pflanzenextrakt wird zur Bildung von Anthocvanin-Bisulfit-Addukten Bisulfit in Form von Natriumbisulfit, schweflige Säure oder SO2-Gas zugesetzt. Anschließend wird der Alkohol entfernt und die ausgefallenen Feststoffe durch Filtration entfernt. Das Anthocyanin-Bisulfit Addukt-Extrakt wird in einer ersten Säule von nicht-komplexierten Flavonoidglykosiden, Pflanzensterolen, Fettsäuren und Triglyceriden gereinigt. Im nächsten Schritt wird der Extrakt angesäuert, um das Gemisch zu desulfitieren, d.h. das Anthocyanin-Bisulfit-Addukt wird zerstört, so dass die Anthocyane wieder frei vorliegen. Anschließend wird das Gemisch in einer zweiten speziellen Säule von Zuckerverbindungen, Salzen und organischen Säuren befreit.

Im zweiten Verfahren wird das Pflanzenmaterial direkt zu Beginn mit saurem Extraktionsmittel behandelt, das ein mit H2SO4 oder HCl versetztes Gemisch aus Ethanol/Wasser oder Methanol/Wasser ist. Der Alkohol wird danach ebenfalls vom Gemisch entfernt, und das Roh-Extrakt anschließend filtriert. Im zweiten Verfahren wird das Gemisch, durch das Fehlen von Anthocyanin-Bisulfit-Addukten, nur einmal mittels einer speziellen Säule von ZuckerVerbindungen, Salzen und organischen Säuren befreit. Das so erhaltene Gemisch kann entsprechend getrocknet werden.

Bei Katarina AA Avikin et. al. ("Phenolic Content and Radical Scavenging Capacity of Berries and related Jams from certificated area in Serbia", Plant Foods for Human Nutrition, Kluwer Academic Publishers, DO, Bd. 64, Nr.3, 26.Mai 2009, S.212-217) handelt es sich um eine wissenschaftliche Abhandlung über den Gehalt von Phenolen, Anthocyanen und freien Ellagsäuren, welche ebenfalls antioxidative Eigenschaften aufweisen, in unterschiedlichen Beerensorten. Es wurde zwischen frischen und eingefroren Beeren (9 Monate bei -18°C) und Beeren in Marmelade, frisch und nach einer Lagerungszeit vergleichen.

Bei der Herstellung, von Marmelade wurden frische Beeren wie bei einer üblichen Marmeladeherstellung mit Zucker verkocht. Ein Teil der frischen Beeren wurde auch kalt-gepresst und der verbliebener Rückstand dann zu Marmelade verarbeitet. Bei der Bestimmung des Gehaltes der Anthocyane in den frischen Beeren wurden diese zerkleinert, mit Methanol versetzt, gerührt und anschließend filtriert und mit HCl in Methanol gewaschen. Aus diesem Filtrat wurde die Menge an Anthocyae, über die Menge von Cyanidin-3-plucoside- chlorid im Filtrat abgeleitet. Zur Bestimmung des Anthocyan-pehaltes in Marmelade wurde die Marmelade analog der frischen Beere behandelt.

Es war deshalb die primäre Aufgabe der vorliegenden Erfindung, ein schonendes Verfahren zum Erzeugen einer Zusammensetzung umfassend die Anthocyane Delphinidin, Cyanidin, Petunidin, Peonidin und Malvidin in glykosidisch gebundener Form anzugeben, wobei das Verhältnis der Gesamtmassen der glykosidisch gebundenen Anthocyane Delphinidin, Cyanidin, Petunidin, Peonidin und Malvidin in der Zusammensetzung im Bereich (3 - 8): (1,8 - 6): (0,5 - 5): 1: (0,5 - 5,5) liegt.

Die zu erzeugende Zusammensetzung sollte dabei gemäß einer ersten Alternative ein Feststoffpulver sein, welches zumindest 25 Gew.-% der genannten Anthocyane umfasst, bezogen auf die Gesamtmasse des Pulvers. Gemäß weiterer Alternativen sollte die zu erzeugende Zusammensetzung mit weiteren (festen oder flüssigen) Bestandteilen so gemischt sein, dass die Gesamtmenge an den besagten Anthocyanen geringer ist.

Es war ergänzend angestrebt, ein Verfahren zum Erzeugen einer Zusammensetzung umfassend die oben genannten Anthocyane anzugeben, bei dem ausgehend von den Früchten von *Vaccinium myrtillus* L. der relative Anteil an frei vorliegenden Zuckern (insbesondere Fructose, andere Monosaccharide sowie Disaccharide) reduziert wird. Die Anwesenheit von Zuckern in zu erzeugenden Zusammensetzungen ist insbesondere aufgrund ihres kariogenen Potenzials und aufgrund ihrer Klebrigkeit nicht erwünscht.

Gemäß einer weiteren Aufgabe der vorliegenden Erfindung sollte die zu erzeugende Zusammensetzung weitere Inhaltsstoffe der Früchte von *Vaccinium myrtillus* L. enthalten, deren biologisch-medizinische Aktivität für einen Konsumenten vorteilhaft ist. Vorzugsweise sollten solche Inhaltsstoffe der Früchte von *Vaccinium myrtillus* L. in der zu erzeugenden Zusammensetzung enthalten sein, welche die Wirkung der oben angegebenen Anthocyane verstärken. Insbesondere war es insoweit angestrebt, die antioxidative Wirkung der Anthocyane zu verstärken, z. B. indem die zu erzeugende Zusammensetzung auf eine solche Weise hergestellt wird, dass aus den Früchten von *Vaccinium myrtillus* L. nicht nur die oben angegebenen Anthocyane, sondern auch weitere Antioxidationsmittel extrahiert, abgetrennt und konzentriert werden. Um die antioxidative Wirkung der angegebenen Anthocyane zu unterstützen, war es dabei angestrebt, die besagten weiteren Antioxidantien in einem bezogen auf die in der Pflanze enthaltenen Mengen an Anthocyanen erhöhtes Mengenverhältnis in der zu erzeugenden Zusammensetzung vorzusehen.

Weitere (Teil-)Aufgaben, die der vorliegenden Erfindung zugrunde liegen, ergeben sich aus der nachfolgenden Beschreibung einschließlich der Beispiele sowie aus den beigefügten Ansprüchen.

Ein erfindungsgemäßes Verfahren zum Erzeugen einer Zusammensetzung umfassend die Anthocyane Delphinidin, Cyanidin, Petunidin, Peonidin und Malvidin in glykosidisch gebundener Form, wobei das Verhältnis der Gesamtmassen der glykosidisch gebundenen Anthocyane Delphinidin, Cyanidin, Petunidin, Peonidin und Malvidin in der Zusammensetzung im Bereich (3 - 8) : (1,8 - 6) : (0,5 - 5) : 1 : (0,5 - 5,5) liegt, ist ein Verfahren mit folgenden Schritten:

    a) Herstellen oder Bereitstellen

(i) eines Saftes oder

(ii) eines Extraktes oder

(iii) sowohl eines Saftes als auch eines Extraktes,
wobei (i) der Saft bzw. (ii) der Extrakt bzw. (iii) Saft und Extrakt jeweils aus den Früchten von *Vaccinium myrtillus* L. hergestellt wird
und

(i) der Saft bzw. (ii) der Extrakt bzw. (iii) die Gesamtheit von Saft und Extrakt sämtliche der Anthocyane Delphinidin, Cyanidin, Petunidin, Peonidin und Malvidin in glykosidisch gebundener Form sowie Zucker umfasst,

b) Trennen (i) des Saftes bzw. (ii) des Extraktes bzw. (iii) sowohl des Saftes als auch des Extraktes, so dass in den Fällen (i) und (ii) eine jeweilige Mischung gebildet wird, die bezogen auf die jeweilige Gesamtmasse an Trockensubstanz an den glykosidisch gebundenen Anthocyanen Delphinidin, Cyanidin, Petunidin, Peonidin und Malvidin angereichert ist und im Fall (iii) zwei Mischungen gebildet werden, von denen zumindest eine bezogen auf die jeweilige Gesamtmasse an Trockensubstanz an den glykosidisch gebundenen Anthocyanen Delphinidin, Cyanidin, Petunidin, Peonidin und Malvidin angereichert ist,
wobei das Verhältnis der Gesamtmassen der glykosidisch gebundenen Anthocyane Delphinidin, Cyanidin, Petunidin, Peonidin und Malvidin in den Fällen (i) und (ii) in der jeweiligen Mischung bzw. im Fall (iii) in der Gesamtheit der zwei Mischungen im Bereich (3 - 8) : (1,8 - 6) : (0,5 - 5) : 1 : (0,5 - 5,5) liegt.

Vorzugsweise liegt das Verhältnis der Gesamtmassen der glykosidisch gebundenen Anthocyane Delphinidin, Cyanidin, Petunidin, Peonidin und Malvidin in den Fällen (i) und (ii) in der jeweiligen Mischung bzw. im Fall (iii) in der Gesamtheit der zwei Mischungen im Bereich (4 - 7,5) : (2,3 - 5,5) : (1 - 4,5) : 1 : (1 - 5).

Besonders bevorzugt liegt das Verhältnis der Gesamtmassen der glykosidisch gebundenen Anthocyane Delphinidin, Cyanidin, Petunidin, Peonidin und Malvidin in den Fällen (i) und (ii) in der jeweiligen Mischung bzw. im Fall (iii) in der Gesamtheit der zwei Mischungen im Bereich (5 - 7) : (2,8 - 5) : (1,5 - 4) : 1 : (1,5 - 4,5).

Die gesamtheit der zwei Mischungen" ist dabei das Produkt einer gedanklichen Vermischung der zwei Mischungen; die "Gesamtheit von Saft und Extrakt" ist das Produkt einer gedanklichen Vermischung von Saft und Extrakt.

Das "Verhältnis der Gesamtmassen der glykosidisch gebundenen Anthocyane Delphinidin, Cyanidin, Petunidin, Peonidin und Malvidin im Fall (iii) in der Gesamtheit der zwei Mischungen" ergibt sich durch Addition der jeweiligen Massen der jeweiligen Anthocyane in den zwei Mischungen zu einer jeweiligen Gesamtmasse und durch anschließende Bildung des Verhältnisses der so ermittelten jeweiligen Gesamtmassen. Beispielsweise wird zunächst die jeweilige Masse von Delphinidin in jeder der zwei Mischungen bestimmt, die beiden so bestimmten Massen werden addiert, so dass sich die Gesamtmasse von Delphinidin ergibt. Diese Gesamtmasse von Delphinidin wird dann ins Verhältnis gesetzt zu den auf analoge Weise bestimmten Gesamtmassen von Cyanidin, Petunidin, Peonidin und Malvidin.

Gemäß einer ersten alternativen Ausgestaltung des erfindungsgemäßen Verfahrens bzw. der bevorzugten Verfahren werden in den Fällen (i) und (ii) die jeweilige Mischung und im Fall (iii) die beiden Mischungen soweit getrocknet, dass eine feste Zusammensetzung erreicht wird. Diese feste Zusammensetzung wird vorzugsweise in ein Pulver überführt, beispielsweise durch Mahlen.

Vorzugsweise umfasst die erzeugte Zusammensetzung (z. B. das Pulver) insgesamt 25 Gew.-% oder mehr, vorzugsweise 30 Gew.-% oder mehr, besonders bevorzugt 36 Gew.-% oder mehr an den genannten glykosidisch gebundenen Anthocyanen, bezogen auf die Gesamtheit der Zusammensetzung.

Bevorzugt ist ein erfindungsmäßiges Verfahren, wobei beim Trennen (i) des Saftes bzw. (ii) des Extraktes bzw. (iii) sowohl des Saftes als auch des Extraktes in Schritt (b), in den Fällen (i) und (ii) eine jeweilige Mischung gebildet wird, die bezogen auf die jeweilige Gesamtmasse an Trockensubstanz (d.h. die Gesamtmasse an Trockensubstanz in einerseits Saft bzw. Extrakt und andererseits in der jeweiligen Mischung nach dem Trennen) an Zuckern abgereichert ist, und im Fall (iii) zwei Mischungen gebildet werden, von denen zumindest eine bezogen auf die jeweilige Gesamtmasse an Trockensubstanz an Zuckern abgereichert ist.

Vorzugsweise ist das Massenverhältnis der oben angegebenen glykosidisch gebundenen Anthocyane in der erzeugten Zusammensetzung zumindest annähernd gleich zu dem Massenverhältnis des in Schritt (a) hergestellten oder bereitgestellten Saftes bzw. Extraktes bzw. der Gesamtheit von Saft und Extrakt. Erfindungsgemäß wird nämlich angestrebt, das natürliche Massenverhältnis, wie es in den Früchten von *Vaccinium myrtillus* L. vorliegt, zumindest im Wesentlichen zu bewahren.

Insoweit versteht es sich, dass in Schnitt (a) vorzugsweise (i) der Saft bzw. (ii) der Extrakt bzw. (iii) Saft und Extrakt jeweils aus den Früchten von *Vaccinium myrtillus* L. hergestellt werden, wobei bevorzugt ausschließlich mechanische und ggf. enzymatische Herstellverfahrensschritte eingesetzt werden, wie zum Beispiel das mechanische Zerkleinern und/oder Pressen der Früchte.

Vorzugsweise enthält somit in Schritt (a) im Fall (i) der Saft bzw. im Fall (ii) der Extrakt bzw. im Fall (iii) die Gesamtheit von Saft und Extrakt sämtliche der Anthocyane Delphinidin, Cyanidin, Petunidin, Peonidin und Malvidin in glykosidisch gebundener Form, wobei das Verhältnis der Gesamtmassen der glykosidisch gebundenen Anthocyane Delphinidin, Cyanidin, Petunidin, Peonidin und Malvidin in (i) dem Saft, (ii) dem Extrakt bzw. (iii) der Gesamtheit von Saft und Extrakt im Bereich (3 - 8) : (1,8 - 6) : (0,5 - 5) : 1 : (0,5 - 5,5) liegt. Das Verhältnis dieser Gesamtmassen wird in Schritt (b) im Wesentlichen bewahrt.

Ein bevorzugtes erfindungsgemäßes Verfahren umfasst den folgenden Schritt:

a) Herstellen (i) eines Saftes oder (ii) eines Extraktes oder (iii) sowohl eines Saftes als auch eines Extraktes, wobei (i) der Saft bzw. (ii) der Extrakt bzw. (iii) Saft und Extrakt jeweils aus Früchten von *Vaccinium myrtillus* L. hergestellt wird bzw. herstellbar ist.

Besonders bevorzugt ist eine Verfahrensgestaltung, bei der in Schritt (a) der Saft bzw. der Extrakt aus Früchten von *Vaccinium myrtillus* L. hergestellt wird. Der Fruchtsaft von *Vaccinium myrtillus* L. umfasst die genannten Anthocyane in den angegebenen Massenverhältnissen; gleiches gilt z. B. für wässrig-ethanolische Extrakte des Tresters von *Vaccinium myrtillus* L. (siehe dazu unten).

Im Rahmen des vorliegenden Textes wird unter Trester der vorwiegend feste Rückstand verstanden, der nach dem Auspressen des Saftes aus Früchten von *Vaccinium myrtillus* L. übrig bleiben.

Im Rahmen der vorliegenden Erfindung war es insbesondere angestrebt, eine Zusammensetzung zu erzeugen, die neben den oben genannten Anthocyanen in dem oben genannten Massenverhältnis zusätzlich auch eine oder mehrere Hydroxybenzoesäuren und/oder eine oder mehrere Hydroxyzimtsäuren in einer solchen Menge umfasst, dass sie aufgrund ihrer antioxidativen Eigenschaften der zu erzeugenden Zusammensetzung einen besonderen Wert verleihen. Früchte von *Vaccinium myrtillus* L. umfassen bereits Hydroxybenzoesäuren (wie Gallussäure) sowie Hydroxyzimtsäuren (wie Sinapinsäure, Ferulasäure, Kaffeesäure, Ellagsäure, Cumarsäure und/oder Chlorogensäure).

Bevorzugt ist somit ein erfindungsgemäßes Verfahren, wobei in Schritt (a) im Fall (i) der Saft bzw. im Fall (ii) der Extrakt bzw. im Fall (iii) die Gesamtheit von Saft und Extrakt sämtliche der Anthocyane Delphinidin, Cyanidin, Petunidin, Peonidin und Malvidin in glykosidisch gebundener Form sowie Zucker
und zusätzlich

- eine oder mehrere Hydroxybenzoesäuren
  oder

- eine oder mehrere Hydroxyzimtsäuren
  oder

- eine oder mehrere Hydroxybenzoesäuren und eine oder mehrere Hydroxyzimtsäuren enthält,

wobei in Schritt (b) in den Fällen (i) und (ii) eine jeweilige Mischung und im Fall (iii) zumindest eine Mischung gebildet wird, welche bezogen auf die jeweilige Gesamtmasse an Trockensubstanz an der oder den Hydroxybenzoesäuren bzw. der oder den Hydroxyzimtsäuren bzw. der oder den Hydroxybenzoesäuren und der oder den Hydroxyzimtsäuren angereichert ist.

Bei einer solchen Verfahrensgestaltung werden somit nicht nur eine Mischung bzw. Mischungen gebildet, die bezogen auf die jeweilige Gesamtmasse an Trockensubstanz an den glykosidisch gebundenen Anthocyanen angereichert sind, sondern die zusätzlich auch an Hydroxybenzoesäure und/oder Hydroxyzimtsäure angereichert sind. Zudem ist die Mischung bzw. sind die Mischungen vorzugsweise an Zuckern abgereichert, siehe dazu oben.

Bei der vorstehend genannten Verfahrensgestaltung ist vorzugsweise die eine oder eine der Hydroxybenzoesäuren Gallussäure.

Zusätzlich oder alternativ ist vorzugsweise die eine oder sind mehrere der Hydroxyzimtsäuren ausgewählt aus der Gruppe bestehend aus Sinapinsäure, Ferulasäure, Kaffeesäure, Ellagsäure, Cumarsäure und/oder Chlorogensäure. Unter den besagten Hydroxyzimtsäuren ist Sinapinsäure bevorzugt.

In einem besonders bevorzugten erfindungsgemäßen Verfahren enthält in Schritt (a) im Fall (i) der Saft bzw. im Fall (ii) der Extrakt bzw. im Fall (iii) die Gesamtheit von Saft und Extrakt
sämtliche der Anthocyane Delphinidin, Cyanidin, Petunidin, Peonidin und Malvidin in glykosidisch gebundener Form sowie Zucker
und zusätzlich Gallussäure und Sinapinsäure,
wobei in Schritt (b) in den Fällen (i) und (ii) eine jeweilige Mischung und im Fall (iii) zumindest eine Mischung gebildet wird, welche bezogen auf die jeweilige Gesamtmasse an Trockensubstanz an Gallussäure und Sinapinsäure angereichert ist.

Es hat sich in eigenen Untersuchungen gezeigt, dass sich die antioxidativen Eigenschaften der Anthocyane, der Gallussäure und der Sinapinsäure synergistisch verstärken.

Bevorzugt ist eine Ausgestaltung des bevorzugten Verfahrens, wobei in Schritt (b) in den Fällen (i) und (ii) eine jeweilige Mischung und im Fall (iii) zumindest eine Mischung gebildet wird, welche bezogen auf die jeweilige Gesamtmasse an Trockensubstanz an den glykosidisch gebundenen Anthocyanen Delphinidin, Cyanidin, Petunidin, Peonidin und Malvidin sowie an Gallussäure und Sinapinsäure angereichert ist, wobei das Verhältnis der Gesamtmassen der glykosidisch gebundenen Anthocyane Delphinidin, Cyanidin, Petunidin, Peonidin und Malvidin sowie von Gallussäure und Sinapinsäure in der Mischung im Bereich (3 - 8) : (1,8 - 6) : (0,5 - 5) : 1 : (0,5 - 5,5) : (0,005 - 1) : (0,00005 - 0,01) liegt.

Besonders bevorzugt liegt das Verhältnis der Gesamtmassen der glykosidisch gebundenen Anthocyane Delphinidin, Cyanidin, Petunidin, Peonidin und Malvidin sowie an Gallussäure und Sinapinsäure in der Mischung im Bereich (4,5 - 7,5) : (2,3 - 5,5) : (1,4 - 4,5) : 1 : (1 - 5) : (0,01 - 0,5) : (0,0001 - 0,005), ganz besonders bevorzugt im Bereich (5 - 7) : (2,8 - 5) : (1,5 - 4) : 1 : (1,5 - 4,5) : (0,05 - 0,2) : (0,0003 - 0,002).

Vorteilhafterweise wird ein solches bevorzugtes Verfahren so ausgestaltet, dass in Schritt (b) in den Fällen (i) und (ii) eine jeweilige Mischung und im Fall (iii) zumindest eine Mischung gebildet wird, welche bezogen auf die jeweilige Gesamtmasse an Trockensubstanz an Gallussäure und/oder Sinapinsäure stärker angereichert ist als an den glykosidisch gebundenen Anthocyanen Delphinidin, Cyanidin, Petunidin, Peonidin und/oder Malvidin. Während somit wie vorstehend ausgeführt, dass Verhältnis der Gesamtmassen der glykosidisch gebundenen Anthocyane sowie von Gallussäure und Sinapinsäure in der Mischung (d. h. nach Schritt (b)) im Bereich (3 - 8) : (1,8 - 6) : (0,5 - 5) : 1 : (0,5 - 5,5) : (0,005 - 1) : (0,00005 - 0,01) liegt, ist es vorteilhaft, wenn das Verfahren so ausgestaltet wird, dass in Schritt (a) im Fall (i) der Saft bzw. im Fall (ii) der Extrakt bzw. im Fall (iii) die Gesamtheit von Saft und Extrakt sämtliche der Anthocyane Delphinidin, Cyanidin, Petunidin, Peonidin und Malvidin in glykosidisch gebundener Form sowie Gallussäure und Sinapinsäure enthält, wobei das Verhältnis der Gesamtmassen der glykosidisch gebundenen Anthocyane Delphinidin, Cyanidin, Petunidin, Peonidin und Malvidin sowie Gallussäure und Sinapinsäure in (i) dem Saft, (ii) dem Extrakt bzw. (iii) der Gesamtheit von Saft und Extrakt im Bereich (3 - 8) : (1,8 - 6) : (0,5 - 5) : 1 : (0,5 - 5,5) : < 0,06 : < 0,001 liegt; hinsichtlich Gallussäure und Sinapinsäure findet eine besondere Anreicherung statt, wobei das jeweilige Verhältnis der Massen von Gallussäure und/oder Sinapinsäure zur Gesamtmasse von Peonidin in (i) dem Saft, (ii) dem Extrakt bzw. (iii) der Gesamtheit von Saft und Extrakt kleiner ist als in den Fällen (i) und (ii) in der jeweiligen Mischung und im Fall (iii) in zumindest einer der Mischungen.

Gemäß dieser besonders bevorzugten Ausgestaltung des erfindungsgemäßen Verfahrens wird also nicht nur (zumindest annähernd) das Verhältnis der Gesamtmassen der glykosidisch gebundenen Anthocyane bei Durchführung des Schrittes (b) bewahrt, sondern es wird gleichzeitig das Verhältnis der Gesamtmassen zugunsten von Gallussäure und Sinapinsäure verschoben. Da vorstehend auch hinsichtlich der Bestandteile Gallussäure und Sinapinsäure Bereiche für Massenanteile angegeben sind, sei hier betont, dass der Massenanteil an Gallussäure und Sinapinsäure bei dieser Verfahrensgestaltung im Vergleich mit den Anthocyanen steigt. Bezogen auf Peonidin (dessen Massenanteil in den obigen Massenverhältnisdefinitionen auf 1 normiert ist) erhöht sich bei Durchführung des Schrittes (b) der Massenanteil an Gallussäure und Sinapinsäure vorzugsweise jeweils zumindest um den Faktor 2, vorzugsweise jeweils um den Faktor 5, besonders bevorzugt jedoch jeweils zumindest den Faktor 20.

Erfindungsgemäß wird in dem Verfahrensschritt (b) (i) der Saft bzw. (ii) der Extrakt bzw. (iii) sowohl der Saft als auch der Extrakt getrennt, so dass in den Fällen (i) und (ii) eine jeweilige Mischung und im Fall (iii) zwei Mischungen gebildet werden, von denen zumindest eine bezogen auf die jeweilige Gesamtmassen an Trockensubstanz eine glykosidisch gebundenen Anthocyanen Delphinidin, Cyanidin, Petunidin, Peonidin und Malvidin angereichert und an Zuckern abgereichert ist. Gemäß einer vorstehend erläuterten bevorzugten Verfahrensgestaltung wird gleichzeitig eine Mischung gebildet, welche bezogen auf die jeweilige Gesamtmasse an Trockensubstanz an der oder den Hydroxybenzoesäuren und/oder der oder den Hydroxyzimtsäuren angereichert ist. Hinsichtlich bevorzugter Hydroxybenzoesäuren bzw. Hydroxyzimtsäuren verweisen wir auf unsere obigen Ausführungen.

Die entsprechende Trennoperation wird der Fachmann in üblicher Weise auswählen. In eigenen Untersuchungen hat es sich überraschenderweise gezeigt, dass die Trennaufgabe besonders effektiv lösbar ist, wenn das erfindungsgemäße Verfahren so gestaltet wird, dass im Schritt (b) das Trennen im Fall (i) des Saftes bzw. im Fall (ii) des Extraktes bzw. im Fall (iii) des Saftes und des Extraktes die folgenden Trennschritte umfasst:

(b) (1) Kontaktieren (i) des Saftes bzw. (ii) des Extraktes bzw. (iii) jeweils des Saftes und des Extraktes mit einem Adsorbens, an das die glykosidisch gebundenen Anthocyane Delphinidin, Cyanidin, Petunidin, Peonidin und Malvidin sowie Hydroxybenzoesäuren und Hydroxyzimtsäuren im Vergleich mit Zuckern selektiv binden,

(b) (2) Desorbieren zumindest der an das Adsorbens gebundenen glykosidisch gebundenen Anthocyane Delphinidin, Cyanidin, Petunidin, Peonidin und Malvidin.

Vorzugsweise werden in Schritt (b) (2) auch Hydroxybenzoesäuren und Hydroxyzimtsäuren desorbiert.

Es versteht sich, dass die beschriebene Ausgestaltung des erfindungsgemäßen Schrittes (b) insbesondere dann vorteilhaft ist, wenn die durchzuführende Trennoperation auf einen Saft und/oder einen Extrakt anzuwenden ist, welcher neben den genannten Anthocyanen auch zusätzlich Gallussäure und Sinapinsäure enthält.

Vorzugsweise erfolgt in Schritt (b) (2) das Desorbieren, indem das Adsorbens mit ein, zwei oder mehr Lösungsmitteln kontaktiert wird, die vorzugsweise ausgewählt sind aus der Gruppe bestehend aus Wasser, Ethanol und deren Mischungen. Als Adsorbens wird der Fachmann insbesondere organische Polymere auswählen, wobei Polymere aus der Gruppe der vernetzten Polystyrole, der Ethylvinylbenzol-Polymere, der aromatischen Polymere (insbesondere Divinylbenzol-Copolymere), der Polyvinylpyrrolidone, der Poly(meth)acrylate (insbesondere der nicht-ionischen aliphatischen Poly(meth)acrylate) bevorzugt sind.

Besonders bevorzugt ist der Einsatz nicht-ionischer vernetzter aromatischer Polymere, bei denen es sich vorzugsweise um Divinylbenzol-Copolymere handelt, sowie der nicht-ionischen aliphatischen Poly(meth)acrylate. Solche Polymere sind im Handel erhältlich. Insbesondere bevorzugt sind die Produkte mit der CAS-Nummern: 37380-43-1, 37380-42-0 und 104219-63-8.

Die eingesetzten Adsorbensmaterialien liegen vorzugsweise als Polymerkügelchen vor; dies ist bei den besagten kommerziell erhältlichen Produkten der Fall.

Vorzugsweise erfolgt in einem erfindungsgemäßen Verfahren Schritt (b) (1), d. h. der Kontaktierungsschritt, bei einem Druck im Bereich von 1 - 3 bar.

Vorzugsweise wird zur Durchführung des Schrittes (b) (1) das Adsorbens in Form einer gepackten Säule eingesetzt. Vergleiche hierzu insgesamt auch die Beispiele weiter unten.

Nach Durchführung des Schrittes (b) liegen in den Fällen (i) und (ii) eine jeweilige Mischung bzw. im Fall (iii) eine Gesamtheit von zwei Mischungen vor, für die das Verhältnis der Gesamtmassen der glykosidisch gebundenen Anthocyane charakteristisch ist (siehe dazu oben). Die primäre Aufgabe der vorliegenden Erfindung ist somit nach Durchführung des Schrittes (b) des erfindungsgemäßen Verfahrens gelöst. Vorzugsweise soll eine zu erzeugende Zusammensetzung jedoch gemäß der oben diskutierten ersten Alternative in Pulverform vorliegen, so dass im Einzelfall bestimmte weitere Verfahrensschritte durchgeführt werden, um die Pulverform zu gewährleisten. Vorteilhaft ist insoweit eine Verfahrensgestaltung, die den folgenden zusätzlichen Schritt umfasst:

(c) Trocknen der nach Schritt (b) vorliegenden Mischung, vorzugsweise bis zu einem Trockensubstanzgehalt von mindestens 80 Gew.-%, bevorzugt bis zu einem Trockensubstanzgehalt von mindestens 90 Gew.-%.

In manchen Fällen wird eine getrocknete Mischung, beispielsweise eine bis auf einen Trockensubstanzgehalt von mindestens 80 Gew.-% (vorzugsweise mindestens 90 Gew.-%) getrocknete Mischung, in einem zusätzlichen Schritt pulverisiert (vorzugsweise gemahlen), um die angestrebte Pulverform zu erreichen.

Vorzugsweise umfasst das nach einem bevorzugten Verfahren hergestellte Pulver einen Gesamtanteil von zumindest 25 Gew.-% an den glykosidisch gebundenen Anthocyanen Delphinidin, Cyanidin, Petunidin, Peonidin und Malvidin, bevorzugt liegt der Gesamtanteil der besagten glykosidisch gebundenen Anthocyane bei zumindest 30 Gew.- %, besonders bevorzugt bei zumindest 36 Gew.-%, jeweils bezogen auf die Gesamtmasse des Pulvers. Zum Erreichen eines solchen Mindestgehaltes an den besagten Anthocyanen wird der Fachmann den Verfahrensschritt (b) so einstellen, dass eine möglichst vollständige Trennung der Anthocyane insbesondere von den im Saft bzw. Extrakt vorhandenen Zuckern erfolgt. Zudem wird der Fachmann (erforderlichenfalls) den Trockensubstanzgehalt der Zusammensetzung durch Durchführung des Schrittes (c) einstellen.

Im erfindungsgemäßen Verfahren wird in Schritt (b) gemäß der Alternative (iii) sowohl ein Saft als auch ein Extrakt hergestellt oder bereitgestellt. Es handelt sich bei dem Saft um Saft aus den Früchten von *Vaccinium myrtillus* L. Der Extrakt ist vorzugsweise ein wässriger oder wässrig-alkoholischer (vorzugsweise wässrig-ethanolischer) Extrakt aus z. B. dem Trester eingesetzter Früchte von *Vaccinium myrtillus* L. Im Rahmen der Alternative (iii) sind sowohl die Inhaltsstoffe des Saftes als auch die des Extraktes wertvoll und sollen jeweils in der zu erzeugenden Zusammensetzung enthalten sein. Ein entsprechendes bevorzugtes Verfahren umfasst demnach die folgenden Schritte:

(a) Herstellen oder Bereitstellen (iii) sowohl eines Saftes als auch eines Extraktes,

(b) Trennen (iii) sowohl des Saftes als auch des Extraktes,

wobei jeweils aus dem Saft eine Mischung resultiert und aus dem Extrakt eine Mischung resultiert, welche jeweils Delphinidin, Cyanidin, Petunidin, Peonidin und Malvidin umfassen, wobei zumindest eine Mischung gebildet wird, welche bezogen auf die jeweilige Gesamtmasse an Trockensubstanz an den Anthocyanen Delphinidin, Cyanidin, Petunidin, Peonidin und Malvidin angereichert und an Zuckern abgereichert ist,

(c) optional Trocknen der nach Schritt (b) vorliegenden Mischungen, vorzugsweise jeweils bis zu einem Trockensubstanzgehalt von mindestens 80 Gew.-%, bevorzugt bis zu einem Trockensubstanzgehalt von mindestens 90 Gew.-%

(d) Vereinen der gemäß Schritt (b) oder gemäß Schritt (c) aus dem Saft resultierenden Mischung mit der aus dem Extrakt resultierenden Mischung,

so dass in der vereinten Mischung das Verhältnis der Gesamtmassen der glykosidisch gebundenen Anthocyane Delphinidin, Cyanidin, Petunidin, Peonidin und Malvidin (3 - 8) : (1,8 - 6) : (0,5 - 5) : 1 : (0,5 - 5,5) ist.

In den Fällen (a) (i) bzw. (a) (iii) ist der hergestellte oder bereitgestellte Saft vorteilhafterweise mittels eines Verfahrens hergestellt bzw. herstellbar, welches das Zerkleinern und Pressen von Früchten von *Vaccinium myrtillus* L. umfasst. Mit anderen Worten handelt es sich vorzugsweise um einen üblichen Fruchtsaft.

Auch der in den Fällen (a) (ii) bzw. (a) (iii) hergestellte oder bereitgestellte Extrakt wird vorzugsweise mittels eines Verfahrens hergestellt bzw. ist mittels eines Verfahrens herstellbar, welches das Zerkleinern und Pressen von Früchten von *Vaccinium myrtillus* L. umfasst. Vorzugsweise ist der in den Fällen (a) (ii) bzw. (a) (iii) hergestellte oder bereitgestellte Extrakt mittels eines Verfahrens hergestellt bzw. ist mittels eines Verfahrens herstellbar, welches die folgenden Schritte umfasst:

- Zerkleinern und Pressen von Früchten von *Vaccinium myrtillus* L., so dass ein Trester erhalten wird

- Extrahieren des erhaltenen Tresters, vorzugsweise mit einem Wasser-Ethanol-Gemisch, und

- optional Filtrieren und/oder Destillieren des erhaltenen Extrakts.

Hinsichtlich bevorzugter Ausgestaltungen eines Verfahrens zum Herstellen eines Extraktes, wie er in den Fällen (a) (ii) bzw. (a) (iii) des erfindungsgemäßen Verfahrens bereitgestellt oder hergestellt wird, sei auf die Beispiele weiter unten verwiesen; es versteht sich, dass in den Beispielen enthaltene Maßnahmen und Parameter im Sinne der vorstehenden allgemeinen Ausführungen variiert oder ersetzt werden können.

Die vorliegende Erfindung betrifft auch eine Zusammensetzung umfassend die glykosidisch gebundenen Anthocyane Delphinidin, Cyanidin, Petunidin, Peonidin und Malvidin sowie Gallussäure und Sinapinsäure, wobei das Verhältnis der Gesamtmassen der glykosidisch gebundenen Anthocyane Delphinidin, Cyanidin, Petunidin, Peonidin und Malvidin sowie von Gallussäure und Sinapinsäure in der Mischung im Bereich (3 - 8) : (1,8 - 6) : (0,5 - 5) : 1 : (0,5 - 5,5) : (0,005 - 1) : (0,00005 - 0,01) liegt.

Eine solche Zusammensetzung liegt vorzugsweise in Pulverform vor. Der Anteil an den genannten glykosidisch gebundenen Anthocyanen in einer solchen vorzugsweise in Pulverform vorliegenden erfindungsgemäßen Zusammensetzung beträgt vorzugsweise zumindest 25 Gew.-%, besonders bevorzugt zumindest 30 Gew.-%, bezogen auf die Gesamtmasse der Zusammensetzung. Es gelten die vorstehend im Zusammenhang mit Produkten des erfindungsgemäßen Verfahrens gemachten Ausführungen entsprechend.

Es hat sich in eigenen Untersuchungen gezeigt, dass sich die antioxidativen Eigenschaften der Anthocyane, der Gallussäure und der Sinapinsäure in einer erfindungsgemäßen Zusammensetzung synergistisch verstärken.

Besonders vorteilhaft sind erfindungsgemäße Zusammensetzungen, deren Zuckergehalt deutlich geringer ist als der Zuckergehalt von bislang bekannten Zusammensetzungen, welche die vorstehend genannten Anthocyane in hoher Konzentration umfassen. Bevorzugt ist der Gesamtanteil an Mono- und Disacchariden in einer erfindungsgemäßen Zusammensetzung kleiner als 2 Gew.-%, vorzugsweise kleiner als 1,5 Gew.-%, bezogen auf die Trockensubstanz der Zusammensetzung.

Vorzugsweise ist eine erfindungsgemäße Zusammensetzung mittels eines erfindungsgemäßen Verfahrens herstellbar, wie es vorstehend offenbart ist. Besonders bevorzugt sind erfindungsgemäße Zusammensetzungen, die nach einem der vorstehend als bevorzugt bezeichneten erfindungsgemäßen Verfahren herstellbar sind.

Die Zusammensetzungen, welche durch das erfindungsgemäße Verfahren erzeugt werden, sowie die erfindungsgemäßen Zusammensetzungen, welche durch die gleichzeitige Anwesenheit glykosidisch gebundener Anthocyane sowie von Gallussäure und Sinapinsäure in einem bestimmten Mischungsverhältnis charakterisiert sind, werden vorzugsweise zur Förderung der Gesundheit eingesetzt. Insbesondere sind solche Zusammensetzungen zur Förderung der Darmgesundheit, zur Förderung der Gesundheit des Herz-Kreislaufsystems, als antiinflammatorische Zusammensetzungen, zur Immunstärkung, zur Förderung der Augengesundheit.

Die vorliegende Erfindung betrifft entsprechend auch die erfindungsgemäßen Zusammensetzungen zur Anwendung in einem Verfahren zur Förderung der Darmgesundheit, zur Förderung der Gesundheit des Herz-Kreislaufsystems, zur Behandlung von Entzündungen, zur Immunstärkung oder zur Förderung der Augengesundheit.

Ein entsprechendes erfindungsgemäßes Verfahren ist ein Verfahren zur Förderung der Darmgesundheit, zur Förderung der Gesundheit des Herz-Kreislaufsystems, der Behandlung von Entzündungen zur Immunstärkung oder zur Förderung der Augengesundheit, mit folgendem Schritt:

Verabreichung einer wirksamen Menge einer Zusammensetzung, die durch das erfindungsgemäße Verfahren erzeugt ist (wie oben beschrieben) oder einer erfindungsgemäßen Zusammensetzung (wie oben beschrieben) an einen zu behandelnden Menschen oder an ein zu behandelndes Tier.

Die vorliegende Erfindung und besonders bevorzugte Ausgestaltungen werden anhand der beigefügten Figur sowie anhand von Beispielen und Bestimmungsmethoden nachfolgend näher erläutert.

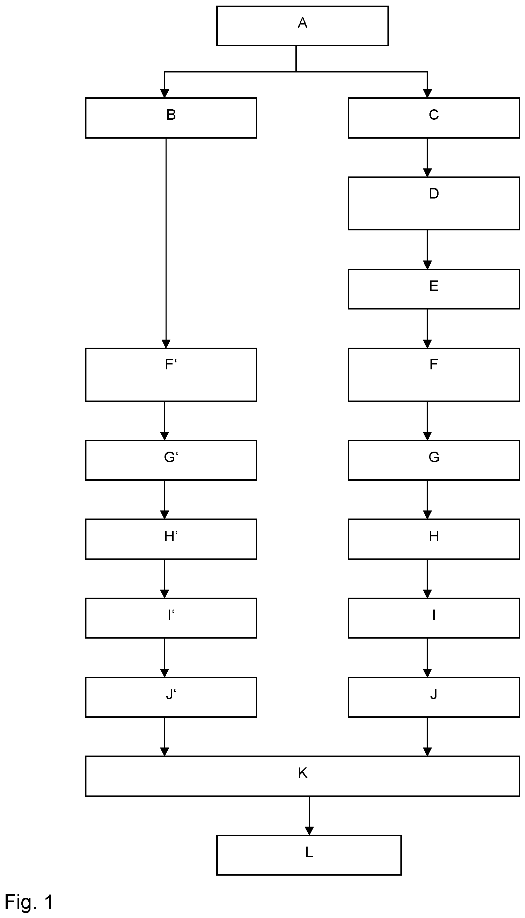

Die beigefügte Figur (Fig. 1) zeigt ein Fließdiagramm, welches die Herstellung einer erfindungsgemäßen Zusammensetzung gemäß Alternative (iii) zeigt.

Gemäß Fig. 1 werden Früchte von *Vaccinium myrtillus* L. bereitgestellt (A). Aus den bereitgestellten Früchten von *Vaccinium myrtillus* L. wird einerseits Saft hergestellt (B) und andererseits ein Trester (C). Dabei werden der Saft und der Trester jeweils mittels eines Verfahrens hergestellt, welches das Zerkleinern und Pressen der Früchte von *Vaccinium myrtillus* L. umfasst. Vorzugsweise wird hierbei ein Enzym (z.B. Cellulase oder Pektinase) zugesetzt, um das Gelieren des Saftes zu verhindern.

Der erhaltene Trester (C) wird gemäß Fig. 1 einem Extraktionsprozess unterworfen (D). Als Extraktionsmittel werden hierbei vorzugsweise Wasser, Ethanol oder Mischungen aus Wasser und Ethanol eingesetzt. Das Extraktionsmittel wird anschließend abdestilliert (E), der Destillationsrückstand umfasst die wertvollen Inhaltsstoffe des Tresters sowie in manchen Fällen Schwebstoffe. Deshalb ist optional gemäß Fig. 1 eine Filtration vorgesehen (F).

Das resultierende Produkt wird nun mit einem Adsorbens kontaktiert, das die glykosidisch gebundenen Anthocyane Delphinidin, Cyanidin, Petunidin, Peonidin und Malvidin sowie Hydroxybenzoesäuren und Hydroxyzimtsäuren (insbesondere Gallussäure und Sinapinsäure) im Vergleich mit Zuckern selektiv bindet (G); diese Vorgehensweise entspricht dem bevorzugten Verfahrensschritt (b) (1) des erfindungsgemäßen Verfahrens. Anschließend werden die an das Adsorbens gebundenen Wertstoffe (insbesondere die besagten Anthocyane sowie Gallussäure und Sinapinsäure; desorbiert (G). Die Desorption wird vorzugsweise erreicht, indem das Adsorbens mit einer wässrig-ethanolischen Lösung behandelt wird. Zu bevorzugten Adsorbens-Materialien siehe oben.

Durch Destillation wird das Desorbat vom Desorptionsmittel (wässrig-ethanolische Lösung) getrennt (H); das resultierende Produkt, welches die Anthocyane sowie Hydroxybenzoesäuren und Hydroxyzimtsäuren (insbesondere Gallussäure und Sinapinsäure) enthält, wird getrocknet (I). Sofern das resultierende getrocknete Produkt noch nicht in Pulverform vorliegt, kann dieses einem optionalen Mahlprozess unterzogen werden (J). Es resultiert ein Pulver, welches die genannten Anthocyane sowie die genannten Hydroxybenzoesäuren und Hydroxyzimtsäuren umfasst. Hinsichtlich bevorzugter Ausgestaltungen der vorstehend geschilderten Maßnahmen siehe oben.

Der gleichzeitig mit dem Trester erzeugte Saft (B) wird nach optionaler Filtration (F') ohne weitere Zwischenschritte mit einem Adsorbens kontaktiert, analog der im Zusammenhang mit Maßnahme (G) diskutierten Vorgehensweise. Es schließen sich weitere Maßnahmen analog den Maßnahmen H, I und (optional) J an. Diese den Saft betreffenden Maßnahmen sind in Figur 1 als G', H', I' und J' bezeichnet.

Die nach den Maßnahmen I, (optional) J bzw. I', (optional) J' vorliegenden Zusammensetzungen (Pulver) sind als solche einsetzbar, vergleiche hierzu die Alternativen (i) und (ii) des erfindungsgemäßen Verfahrens. Gemäß Alternative (iii) ist zumindest eine der nach Schritt I, (optional) J bzw. I', (optional) J' vorliegenden Mischungen bezogen auf die jeweilige Gesamtmasse an Trockensubstanz an den glykosidisch gebundenen Anthocyanen Delphinidin, Cyanidin, Petunidin, Peonidin und Malvidin angereichert und an Zuckern abgereichert.

Gemäß Figur 1 und im Einklang mit einer bevorzugten Ausgestaltung eines erfindungsgemäßen Verfahrens werden die genannten resultierenden Mischungen miteinander vereint (vermischt, verschnitten) (K) es resultiert eine erfindungsgemäße Zusammensetzung (L).

Bestimmungsmethoden:

Bestimmung des Trockensubstanzanteils:

Zur Durchführung der Bestimmung werden folgende Geräte benötigt: Analysenwaage (± 0,001 g), Exsikkator, Metallschale, Spatel, Tiegelzange und Trockenschrank.

Die Metallschale wird vor der Untersuchung eine Stunde lang bei 110°C getrocknet und im Exsikkator auf Raumtemperatur abgekühlt. Das Tara der Metallschale wird ermittelt. Anschließend werden 5g der zu untersuchenden Substanz genau eingewogen, wobei die Substanz in der Metallschale gleichmäßig verteilt wird, so dass eine möglichst große Oberfläche entsteht, um einen optimalen Trocknungsprozess zu gewährleisten. Die Probe wird bis zur Gewichtskonstanz in einen auf 110°C thermostatisierten Trockenschrank gestellt, im Exsikkator auf Raumtemperatur abgekühlt und gewogen. Gewichtskonstanz liegt vor, wenn die gewogene Probe nach weiteren zwei Stunden in einem auf 110°C thermostatisieren Trockenschrank und Abkühlung im Exsikkator auf Raumtemperatur ein Gewicht besitzt, welches maximal um 2 mg niedriger ist als das zunächst gewogene Gewicht.

Sofern es sich bei der zu untersuchenden Substanz um eine Flüssigkeit handelt, wird die Substanz in einer mit Glasfasern gefüllten Metallschale verteilt. Es wird das Tara der Metallschale inklusive Glasfasern bestimmt. Vor der Probenaufgabe wird die mit Glasfasern gefüllte Metallschale bis zur Gewichtskonstanz getrocknet und im Exsikkator auf Raumtemperatur abgekühlt.

Der Trockensubstanzanteil wird nach folgender Gleichung bestimmt:

$$TR[Gew.\text{-}\%] = \frac{a * 100}{m}$$

wobei gilt:

TR = Trockensubstanzanteil in Gewichtsprozenten [Gew.-%]

a = Gewicht des Trockensubstanzanteils in Gramm [g]

m = Substanzeinwaage in Gramm [g]

Bestimmung der Verhältnisse der Gesamtmassen der glykosidisch gebundenen Anthocyane Delphinidin, Cyanidin, Petunidin, Peonidin und Malvidin:

Die Bestimmung erfolgt gemäß European Pharmacopoeia 6.4, Monographie betreffend "Fresh bilberry fruit dry extract, refind and standardized" (07/2008: 2394 corrected 6.4), Tests, anthocyanidins.

Hierbei ist zu berücksichtigen, dass gemäß Monographie der Anteil an "total anthocyanidins" bestimmt wird. Abweichend davon wird zum Zweck der Bestimmung des Verhältnisses der Gesamtmassen der besagten glykosidisch gebundenen Anthocyane die jeweilige Gesamtmasse der einzelnen glykosidisch gebundenen Anthocyane bestimmt, wobei jeweils die Massen der unterschiedlichen Glykoside (Galactosid; Arabinosid; Glucosid) addiert werden.

Bestimmung des Gesamtzuckergehalts:

Die Bestimmung des Gesamtgehaltes an Mono- und Disacchariden erfolgt gemäß der Vorschrift auf Seite 118ff des Buches "Reinhard Matissek / Gabriele Steiner / Frank-M. Schnepel: Lebensmittelanalytik: Grundzüge, Methoden, Anwendungen, Berlin, Springer-Verlag, 1992". Der Gesamtzuckergehalt entspricht der Summe aller quantitativ bestimmten Zuckermengen.

Bestimmung des Gehalts an Sinapin- und/oder Gallussäure:

Die Bestimmung des Gehaltes an Sinapin- und/oder Gallussäure im Saft, Trester oder Extrakt wird per Eco-LC-MS[2] durchgeführt. Dazu werden die Proben (0,2 - 2,0 g) genau in einen Messkolben eingewogen, mit dem internen Standard Vanillin (0,1 g) versetzt, mit Ethanol auf eine definierte Masse aufgewogen und über einen Mikrofilter (Porengröße ca. 0,45 $\mu$m) vor der Analyse filtriert. Ein Volumen von 0,2 $\mu$l der so vorbereiteten Probe wird in ein LC/MS System (QTrap), gekoppelt mit einem PDA-Detektor, injiziert. Als stationäre Phase dient "fused core" Säulenmaterial (Poroshell 120 SC-C18, 2,7 $\mu$m, 2,1 mm x 50 mm). Die Säule wird auf eine Temperatur von 50°C temperiert. Die mobile Phase besteht aus 0,1% Ameisensäure in Wasser (A) und Ethanol (B) und der Fluss beträgt 0,3 ml/min. Der Gradient der mobilen Phase kann der Tabelle 1 entnommen werden:

Tabelle 1: Gradient mobile Phase

| Zeit [min] | A [Vol.-%] | B [Vol.-%] |
|---|---|---|
| 0,00 | 95 | 5 |
| 15,00 | 5 | 95 |
| 20,00 | 5 | 95 |
| 21,00 | 95 | 5 |

(fortgesetzt)

| Zeit [min] | A [Vol.-%] | B [Vol.-%] |
|---|---|---|
| 25,00 | 95 | 5 |

Die qualitative Analyse erfolgt über den Vergleich mit Referenzspektren (Massen-). Diese umfassen einerseits die massenspektrometrischen Daten sowie teilweise auch UV-Spektren. Durch die Elutionsposition des Peaks erfolgt eine Untermauerung.

Tabelle 2: Daten Sinapinsäure, Gallussäure und Vanillin

| Name | Mol. Masse [g/mol] | MRM1 | MRM2 | CE1 [eV] | CE2 [eV] | Retentionszeit [min] | LOD [ppm] |
|---|---|---|---|---|---|---|---|
| Gallussäure | 170 | 168,8-124,9 | 168,8-78,9 | -20 | -34 | 0,7 | 0,1 |
| Sinapinsäure | 224 | 223,1-120,9 | 223,1-164,0 | -38 | -20 | 4,6 | 0,1 |
| Vanillin | 158 | 157,0-141,9 | 157,0-96,9 | -18 | -28 | 3,3 | -- |

Abkürzungen: MRM = Multiple Reaction Monitoring, CE = collision energy, LOD = Limit of Detection

Die quantitative Bestimmung erfolgt mit Hilfe einer externen Kalibrierfunktionen und über den internen Standard. Durch eine Kalibrierung mit Referenzsubstanzen im erwarteten Konzentrationsbereich wird eine Regressionsgerade erhalten. Dabei werden die Flächen der Massenspuren integriert und gegen den jeweiligen Gehalt an Referenzsubstanz aufgetragen. Mit der erhaltenen Funktionsgleichung der Regressionsgeraden (linear) erfolgt die Berechnung des Gehaltes der jeweiligen Substanz in der Probe unter Berücksichtigung der Einwaage.

Beispiele:

Beispiel 1: Herstellen eines Bilberry-Tresters und eines Bilberry-Saftes aus Bilberry-Früchten (Vaccinium myrtillus L.):

10t Bilberry-Früchte (Früchte von Vaccinium myrtillus L.) mit einer Trockensubstanz von 12 Gew.-% und einem Gehalt an glykosidisch gebundenen Anthocyanen von 0,30 Gew.-% wurden nach Zugabe von Pektinasen zerkleinert und ausgepresst. Hierdurch entstanden einerseits ca. 1,3t Bilberry-Trester mit einer Trockensubstanz von 40 Gew.-% und einem Gehalt an glykosidisch gebundenen Anthocyanen von 0,8 Gew.-% sowie anderseits ca. 8,7t Bilberry-Saft mit einer Trockensubstanz von 8,0 Gew.-% und einem Gehalt an glykosidisch gebundenen Anthocyanen von 0,22 Gew.-%. Alle Gewichtsprozentangaben beziehen sich auf die Gesamtmasse der jeweiligen Fraktion (Bilberry-Trester bzw. Bilberry-Saft).

Das Verhältnis der Gesamtmassen der glykosidisch gebundenen Anthocyane Delphinidin, Cyanidin, Petunidin, Peonidin und Malvidin sowie von Gallussäure und Sinapinsäure in dem Bilberry-Trester liegt bei 5,9 : 4,1 : 2,4 : 1,0 : 1,9 : 0,03 : <0,0001. Der Gesamtzuckergehalt (Fructose und andere Zucker) im Bilberry-Trester liegt bei Gew.-%.

Das Verhältnis der Gesamtmassen der glykosidisch gebundenen Anthocyane Delphinidin, Cyanidin, Petunidin, Peonidin und Malvidin sowie von Gallussäure und Sinapinsäure in dem Bilberry-Saft liegt bei 4,0 : 3,4 : 2,0 : 1,0 : 2,3 : 0,04 : <0,0001. Der Gesamtgehalt an Mono- und Disacchariden im Bilberry-Saft liegt bei Gew.-%.

Die Herstellung des Bilberry-Saftes entspricht Schritt (a) (i) bzw. (iii) des erfindungsgemäßen Verfahrens.

Beispiel 2: Herstellen eines Extraktes aus dem Bilberry-Trester gemäß Beispiel 1 (entsprechend Schritt (a) des erfindungsgemäßen Verfahrens (Alternative (ii) bzw. (iii)):

1100 kg des nach Beispiel 1 hergestellten Bilberry-Tresters wurden in einer Perkolationsapparatur mit einem Volumen von 3000 l bei einer Temperatur von 35 bis 40 °C extrahiert. Die Extraktion wurde 4-mal mit jeweils 2,5t einer Wasser-Ethanol-Lösung (30 Vol.-% Ethanol), dessen pH-Wert auf 2,7 (±0,2) eingestellt wurde, durchgeführt. Aus dem erhaltenen Vorextrakt wurde bei einer maximalen Sumpftemperatur von 40 °C bei reduziertem Druck und einem Durchlauf von ca. 200 l/h - 400 l/h das Ethanol abdestilliert und anschließend wurde die verbleibende Lösung mittels weiterer Destillation auf einen Trockensubstanzgehalt von 6,0 Gew.-% eingestellt. Der Vorextrakt wurde mit vollentsalztem Wasser auf eine Trockensubstanz von 3,5 Gew.-% verdünnt und mit Hilfe eines Separators wurden nichtlösliche Feststoffe abfiltriert. Das erhaltene Produkt wird als Extrakt bezeichnet und in Beispiel 3 weiter eingesetzt.

Beispiel 3: Trennen des Extraktes aus Beispiel 2 (entsprechend Schritt (b) eines bevorzugten erfindungsgemäßen Verfahrens (Alternative (ii) bzw. (iii)):

Ein zylindrischer Stahlkörper, der von beiden Seiten mit einem Flansch verschlossen ist, wurde als Säule verwendet. Das Säulenvolumen betrug 1200 l. In beiden Flanschen war jeweils ein Rohr angebracht, durch den Flüssigkeiten in die Säule hinein bzw. aus der Säule herausgepumpt werden konnten. Die Säule war mit einem vernetzten Polymethacrylharz (CAS-Nummer:37380-43-1) als Adsorbens aufgefüllt.

Der Extrakt aus Beispiel 2 wurde bei einem Volumenstrom von ca. 5000 l/h mit dem Adsorbens kontaktiert und anschließend wurde die Säule mit 3600 l (3 Säulenvolumina) Wasser gespült. Der Volumenstrom wurde dabei bei ca. 5000 l/h und die Temperatur innerhalb der Säule bei 34°C bis 39°C gehalten. Der Säulendruck lag im Bereich von 1,0 bis 3,0 bar.

Die auf dem Säulenmaterial adsorbierten Inhaltsstoffe des Extraktes wurden anschließend mit 2000 l (1,66 Säulenvolumina) einer Wasser-Ethanol-Lösung (40 Vol.-% Ethanol), dessen pH-Wert auf 2,7 ($\pm$0,2) eingestellt war, desorbiert. Das erhaltene Gemisch aus Wasser, Ethanol und desorbierten Inhaltsstoffen des Extraktes wurde bei einer Sumpftemperatur von maximal 40 °C bei reduziertem Druck bis zu einem Trockensubstanzgehalt von 54 Gew.-% aufdestilliert. Das aufdestillierte Produkt wird als Mischung bezeichnet und in Beispiel 4 weiter eingesetzt.

Beispiel 4: Trocknen der Mischung aus Beispiel 3 (entsprechend Schritt (c) eines bevorzugten erfindungsgemäßen Verfahrens):

Die Mischung aus Beispiel 3 wurde bei max. 60°C und einem Druck von < 100 mbar bis auf einen Trockengehalt von 2,0 Gew.-% getrocknet. Die getrocknete Mischung wurde in einer Bohle-Mühle auf eine Partikelgröße < 600 $\mu$m gemahlen.

Es wurden 7,7 kg Pulver mit einer Trockensubstanz von 98,0 Gew.-% und einem Anthocyangehalt von 26 Gew.-% erhalten.

Das Verhältnis der Gesamtmassen der glykosidisch gebundenen Anthocyane Delphinidin, Cyanidin, Petunidin, Peonidin und Malvidin sowie von Gallussäure und Sinapinsäure in dem Pulver liegt bei 5,9 : 3,9 : 3,0 : 1,0 : 2,5 : 0,17 : 0,0006. Der Gesamtgehalt an Mono- und Disacchariden im Pulver liegt bei 0,9 Gew.-%.

Beispiel 5: Trennen des Bilberry-Saftes aus Beispiel 1 (entsprechend Schritt (b) des erfindungsgemäßen Verfahrens (Alternative (i) bzw. (iii)):

Die Trennung des Bilberry-Saftes aus Beispiel 1 erfolgte analog zu der Trennung in Beispiel 3. Es wurden 2000 l des nach Beispiel 1 hergestellten Bilberry-Saftes statt des Extraktes aus Beispiel 2 verwendet. Das aufdestillierte Produkt wird als Mischung bezeichnet und in Beispiel 6 weiter eingesetzt.

Beispiel 6: Trocknen der Mischung aus Beispiel 5 (entsprechend Schritt (c) eines bevorzugten erfindungsgemäßen Verfahrens):

Die Trocknung der Mischung aus Beispiel 5 erfolgte analog zu der Trennung in Beispiel 4. Es wurden 7,4 kg Pulver mit einer Trockensubstanz von 98,0 Gew.-% und einem Anthocyangehalt von 26 Gew.-% erhalten. Das Verhältnis der Gesamtmassen der glykosidisch gebundenen Anthocyane Delphinidin, Cyanidin, Petunidin, Peonidin und Malvidin sowie von Gallussäure und Sinapinsäure in dem Pulver liegt bei 5,0 : 2,9 : 2,2 : 1 : 1,7 : 0,09 : 0,0019. Der Gesamtgehalt an Mono- und Disacchariden im Pulver liegt bei 0,9 Gew.-%.

Beispiel 7: Vereinen der gemäß Beispiel 4 und Beispiel 6 hergestellten Pulver (entsprechend Schritt (d) eines bevorzugten erfindungsgemäßen Verfahrens):

7,0 kg des nach Beispiel 4 hergestellten Pulvers und 7,0 kg des nach Beispiel 6 hergestellten Pulvers wurden vermischt. Das Verhältnis der Gesamtmassen der glykosidisch gebundenen Anthocyane Delphinidin, Cyanidin, Petunidin, Peonidin und Malvidin sowie von Gallussäure und Sinapinsäure in der Pulvermischung liegt bei 5,4 : 3,4 : 2,6 : 1 : 2,1 : 0,13 : 0,0013. Der Gesamtgehalt an Mono- und Disacchariden in der Pulvermischung liegt bei 0,9 Gew.-%.

**Patentansprüche**

1. Verfahren zum Erzeugen einer Zusammensetzung umfassend die Anthocyane Delphinidin, Cyanidin, Petunidin, Peonidin und Malvidin in glykosidisch gebundener Form, wobei das Verhältnis der Gesamtmassen der glykosidisch gebundenen Anthocyane Delphinidin, Cyanidin, Petunidin, Peonidin und Malvidin in der Zusammensetzung im Bereich (3 - 8): (1,8 - 6): (0,5 - 5): 1: (0,5 - 5,5) liegt, mit folgenden Schritten:

(a) Herstellen oder Bereitstellen

(i) eines Saftes oder
(ii) eines Extraktes oder
(iii) sowohl eines Saftes als auch eines Extraktes,

wobei (i) der Saft bzw. (ii) der Extrakt bzw. (iii) Saft und Extrakt aus Früchten von *Vaccinium myrtillus* L. hergestellt wird
und
(i) der Saft bzw. (ii) der Extrakt bzw. (iii) die Gesamtheit von Saft und Extrakt sämtliche der Anthocyane Delphinidin, Cyanidin, Petunidin, Peonidin und Malvidin in glykosidisch gebundener Form sowie Zucker umfasst,
(b) Trennen (i) des Saftes bzw. (ii) des Extraktes bzw. (iii) sowohl des Saftes als auch des Extraktes, so dass in den Fällen (i) und (ii) eine jeweilige Mischung gebildet wird, die bezogen auf die jeweilige Gesamtmasse an Trockensubstanz an den glykosidisch gebundenen Anthocyanen Delphinidin, Cyanidin, Petunidin, Peonidin und Malvidin angereichert ist und im Fall (iii) zwei Mischungen gebildet werden, von denen zumindest eine bezogen auf die jeweilige Gesamtmasse an Trockensubstanz an den glykosidisch gebundenen Anthocyanen Delphinidin, Cyanidin, Petunidin, Peonidin und Malvidin angereichert ist,

wobei das Verhältnis der Gesamtmassen der glykosidisch gebundenen Anthocyane Delphinidin, Cyanidin, Petunidin, Peonidin und Malvidin in den Fällen (i) und (ii) in der jeweiligen Mischung bzw. im Fall (iii) in der Gesamtheit der zwei Mischungen im Bereich (3 - 8): (1,8 - 6): (0,5 - 5): 1: (0,5 - 5,5) liegt.

2. Verfahren nach Anspruch 1, wobei beim Trennen (i) des Saftes bzw. (ii) des Extraktes bzw. (iii) sowohl des Saftes als auch des Extraktes in Schritt (b), in den Fällen (i) und (ii) eine jeweilige Mischung gebildet wird, die bezogen auf die jeweilige Gesamtmasse an Trockensubstanz an Zuckern abgereichert ist, und im Fall (iii) zwei Mischungen gebildet werden, von denen zumindest eine bezogen auf die jeweilige Gesamtmasse an Trockensubstanz an Zuckern abgereichert ist.

3. Verfahren nach Anspruch 1 oder 2, wobei in Schritt (a) im Fall (i) der Saft bzw. im Fall (ii) der Extrakt bzw. im Fall (iii) die Gesamtheit von Saft und Extrakt sämtliche der Anthocyane Delphinidin, Cyanidin, Petunidin, Peonidin und Malvidin in glykosidisch gebundener Form enthält, wobei das Verhältnis der Gesamtmassen der glykosidisch gebundenen Anthocyane Delphinidin, Cyanidin, Petunidin, Peonidin und Malvidin in (i) dem Saft, (ii) dem Extrakt bzw. (iii) der Gesamtheit von Saft und Extrakt im Bereich (3 - 8) : (1,8 - 6) : (0,5 - 5) : 1 : (0,5 - 5,5) liegt.

4. Verfahren einem der Ansprüche 1 bis 3, wobei in Schritt (a) im Fall (i) der Saft bzw. im Fall (ii) der Extrakt bzw. im Fall (iii) die Gesamtheit von Saft und Extrakt sämtliche der Anthocyane Delphinidin, Cyanidin, Petunidin, Peonidin und Malvidin in glykosidisch gebundener Form sowie Zucker
und zusätzlich

- eine oder mehrere Hydroxybenzoesäuren
oder
- eine oder mehrere Hydroxyzimtsäuren
oder
- eine oder mehrere Hydroxybenzoesäuren und eine oder mehrere Hydroxyzimtsäuren

enthält,
wobei in Schritt (b) in den Fällen (i) und (ii) eine jeweilige Mischung und im Fall (iii) zumindest eine Mischung gebildet wird, welche bezogen auf die jeweilige Gesamtmasse an Trockensubstanz an der oder den Hydroxybenzoesäuren bzw. der oder den Hydroxyzimtsäuren bzw. der oder den Hydroxybenzoesäuren und der oder den Hydroxyzimtsäuren angereichert ist.

5. Verfahren nach Anspruch 4, wobei
die eine oder eine der Hydroxybenzoesäuren Gallussäure ist
und/oder
die eine oder mehrere Hydroxyzimtsäuren ausgewählt sind aus der Gruppe bestehend aus Sinapinsäure, Ferulasäure, Kaffeesäure, Ellagsäure, Cumarsäure und/oder Chlorogensäure.

6. Verfahren nach einem der vorangehenden Ansprüche, wobei in Schritt (a) im Fall (i) der Saft bzw. im Fall (ii) der Extrakt bzw. im Fall (iii) die Gesamtheit von Saft und Extrakt
sämtliche der Anthocyane Delphinidin, Cyanidin, Petunidin, Peonidin und Malvidin in glykosidisch gebundener Form sowie Zucker
und zusätzlich Gallussäure und Sinapinsäure enthält,
wobei in Schritt (b) in den Fällen (i) und (ii) eine jeweilige Mischung und im Fall (iii) zumindest eine Mischung gebildet wird, welche bezogen auf die jeweilige Gesamtmasse an Trockensubstanz an Gallussäure und Sinapinsäure angereichert ist.

7. Verfahren nach Anspruch 6, wobei in Schritt (b) in den Fällen (i) und (ii) eine jeweilige Mischung und im Fall (iii) zumindest eine Mischung gebildet wird, welche bezogen auf die jeweilige Gesamtmasse an Trockensubstanz an den glykosidisch gebundenen Anthocyanen Delphinidin, Cyanidin, Petunidin, Peonidin und Malvidin sowie an Gallussäure und Sinapinsäure angereichert ist, wobei das Verhältnis der Gesamtmassen der glykosidisch gebundenen Anthocyane Delphinidin, Cyanidin, Petunidin, Peonidin und Malvidin sowie von Gallussäure und Sinapinsäure in der Mischung im Bereich (3 - 8) : (1,8 - 6) : (0,5 - 5) : 1 : (0,5 - 5,5) : (0,005 - 1) : (0,00005 - 0,01) liegt.

8. Verfahren nach Anspruch 7, wobei in Schritt (a) im Fall (i) der Saft bzw. im Fall (ii) der Extrakt bzw. im Fall (iii) die Gesamtheit von Saft und Extrakt sämtliche der Anthocyane Delphinidin, Cyanidin, Petunidin, Peonidin und Malvidin in glykosidisch gebundener Form sowie Gallussäure und Sinapinsäure enthält, wobei das Verhältnis der Gesamtmassen der glykosidisch gebundenen Anthocyane Delphinidin, Cyanidin, Petunidin, Peonidin und Malvidin sowie Gallussäure und Sinapinsäure in (i) dem Saft, (ii) dem Extrakt bzw. (iii) der Gesamtheit von Saft und Extrakt im Bereich (3 - 8) : (1,8 - 6) : (0,5 - 5) : 1 : (0,5 - 5,5) : < 0,06 : < 0,001 liegt.

9. Verfahren nach einem der vorangehenden Ansprüche, wobei in Schritt (b) das Trennen im Fall (i) des Saftes bzw. im Fall (ii) des Extraktes bzw. im Fall (iii) des Saftes und des Extraktes die folgenden Trennschritte umfasst:

(b) (1) Kontaktieren (i) des Saftes bzw. (ii) des Extraktes bzw. (iii) jeweils des Saftes und des Extraktes mit einem Adsorbens, an das die glykosidisch gebundenen Anthocyane Delphinidin, Cyanidin, Petunidin, Peonidin und Malvidin sowie Hydroxybenzoesäuren und Hydroxyzimtsäuren im Vergleich mit Zuckern selektiv binden,
(b) (2) Desorbieren zumindest der an das Adsorbens gebundenen glykosidisch gebundenen Anthocyane Delphinidin, Cyanidin, Petunidin, Peonidin und Malvidin.

10. Verfahren nach Anspruch 9, wobei in Schritt (b) (2) auch Hydroxybenzoesäuren und Hydroxyzimtsäuren desorbiert werden.

11. Verfahren nach Anspruch 9 oder 10, wobei in Schritt (b) (2) das Desorbieren erfolgt, indem das Adsorbens mit ein, zwei oder mehr Lösungsmitteln kontaktiert wird, die vorzugsweise ausgewählt sind aus der Gruppe bestehend aus Wasser, Ethanol und deren Mischungen.

12. Verfahren nach einem der vorangehenden Ansprüche, mit folgenden Schritten:

(a) Herstellen oder Bereitstellen (iii) sowohl eines Saftes als auch eines Extraktes,
(b) Trennen (iii) sowohl des Saftes als auch des Extraktes,

wobei jeweils aus dem Saft eine Mischung resultiert und aus dem Extrakt eine Mischung resultiert, welche jeweils Delphinidin, Cyanidin, Petunidin, Peonidin und Malvidin umfassen, wobei zumindest eine Mischung gebildet wird, welche bezogen auf die jeweilige Gesamtmasse an Trockensubstanz an den Anthocyanen Delphinidin, Cyanidin, Petunidin, Peonidin und Malvidin angereichert und an Zuckern abgereichert ist,

(c) optional Trocknen der nach Schritt (b) vorliegenden Mischungen, vorzugsweise jeweils bis zu einem Trockensubstanzgehalt von mindestens 80 Gew.-%, bevorzugt bis zu einem Trockensubstanzgehalt von mindestens 90 Gew.-%
(d) Vereinen der gemäß Schritt (b) oder gemäß Schritt (c) aus dem Saft resultierenden Mischung mit der aus dem Extrakt resultierenden Mischung,

so dass in der vereinten Mischung das Verhältnis der Gesamtmassen der glykosidisch gebundenen Anthocyane Delphinidin, Cyanidin, Petunidin, Peonidin und Malvidin (3 - 8) : (1,8 - 6) : (0,5 - 5) : 1 : (0,5 - 5,5) ist.

13. Zusammensetzung umfassend die glykosidisch gebundenen Anthocyane Delphinidin, Cyanidin, Petunidin, Peonidin und Malvidin sowie Gallussäure und Sinapinsäure, wobei das Verhältnis der Gesamtmassen der glykosidisch gebundenen Anthocyane Delphinidin, Cyanidin, Petunidin, Peonidin und Malvidin sowie von Gallussäure und Sinapinsäure in der Mischung im Bereich (3 - 8) : (1,8 - 6) : (0,5 - 5) : 1 : (0,5 - 5,5) : (0,005 - 1) : (0,00005 - 0,01) liegt.

14. Zusammensetzung nach Anspruch 13, wobei der Gesamtanteil an Mono- und Disacchariden kleiner ist als 2 Gew.-%, vorzugsweise kleiner als 1,5 Gew.-%, bezogen auf die Trockensubstanz der Zusammensetzung.

15. Zusammensetzung nach Anspruch 13 oder 14, herstellbar mittels eines Verfahrens nach einem der Ansprüche 1 bis 12.

## Claims

1. A process for the production of a composition comprising anthocyans of delphinidine, cyanidine, petunidine, peonidine and malvidine type in its glyosidic form, wherein the ratio of the total masses of said anthocyans of delphinidine, cyanidine, petunidine, peonidine and malvidine type in its glyosidic form within said composition ranges from (3-8):(1.8:6):(0.5-5):1:(0.5-5.5) encompassing the following steps:

    (a) making or providing

        (i) a juice or
        (ii) an extract or
        (iii) a juice as well as an extract

    wherein (i) said juice or respectively (ii) said extract or (iii) said total of juice and extract is produced from fruits of *Vaccinium myrtillus L,* and
    (i) said juice or respectively (ii) said extract or (iii) the total of juice and extract comprises all anthocyans of delphinidine, cyanidine, petunidine, peonidine and malvidine type in its glyosidic form and sugar,
    (b) separating (i) said juice or respectively (ii) said extract or (iii) said total of juice and extract, in order to obtain in case of (i) and (ii) a respective mixture enriched in said anthocyans of delphinidine, cyanidine, petunidine, peonidine and malvidine type in its glyosidic form - calculated on the respective total masses of dry matter - and in case of (iii) to obtain two mixtures from which at least one is enriched in said anthocyans of delphinidine, cyanidine, petunidine, peonidine and malvidine type in its glyosidic form,
    wherein the ratio of the total masses of said anthocyans of delphinidine, cyanidine, petunidine, peonidine and malvidine type in its glyosidic form in case of (i) and (ii) within the respective mixture and in case of (iii) in the total of both mixtures ranges from (3-8):(1.8:6):(0.5-5):1:(0.5-5.5)

2. The process of claim 1, wherein in the course of the separation (i) of the juice or respectively (ii) the extract or (iii) the total of juice and extract in step (b) in case of (i) and (ii) a respective mixture is formed, depleted of sugars - calculated on the respective total mass of dry matter - and in case of (iii) two mixtures are formed, of which at least one is depleted of sugars - calculated on the respective total mass of dry matter.

3. The process of claim 1 or 2, wherein in step (a) in case of (i) the juice and in case of (ii) the extract and in case of (iii) the total of juice and extract comprises all anthocyans of delphinidine, cyanidine, petunidine, peonidine and malvidine type in its glyosidic form, wherein the ratio of the total masses of said anthocyans of delphinidine, cyanidine, petunidine, peonidine and malvidine type in its glyosidic form in (i) said juice, (ii) said extract or (iii) said total of juice extract ranges from (3-8):(1.8:6):(0.5-5):1:(0.5-5.5).

4. The process of one of claims 1 to 3, wherein in step (a) in case of (i) the juice or respectively in case of (ii) the extract or in case of (iii) the total of juice and extract comprise all anthocyans of delphinidine, cyanidine, petunidine, peonidine and malvidine type in its glyosidic form, and additionally

    - one or more hydroxy benzoic acid ester; or
    - one or more hydroxy cinnamic acid, or

- one or more hydroxy benzoic acid ester and one or more hydroxy cinnamic acid,

wherein in step (b) in case of (i) or (ii) a respective mixture and in case of (iii) at least one mixture is formed, which - calculated on the total mass of dry matter - is enriched in one or more hydroxy benzoic acid ester or one or more hydroxy cinnamic acid, or one or more hydroxy benzoic acid ester and one or more hydroxy cinnamic acid.

5. The process of Claim 4, wherein
said one or more hydroxy benzoic acid is gallic acid and/or
said one or more hydroxy cinnamic acid is selected from the group consisting of sinapic acid, ferulic acid, caffeic acid, ellagic acid, cumaric acid and/or chlorogenic acid.

6. The process of any of the preceding claims, wherein in step (a) in case of (i) the juice or respectively in case of (ii) the extract or in case of (iii) the total of juice and extract contains
all anthocyans of delphinidine, cyanidine, petunidine, peonidine and malvidine type in its glyosidic form and sugar, and additionally
gallic acid and sinapic acid,
wherein in step (b) in case of (i) and (ii) a respective mixture and in case of (iii) at least one mixture is formed, which is enriched in gallic acid and sinapic acid - calculated on the respective total mass of dry matter.

7. The process of claim 6, wherein in step (b) in case of (i) and (ii) a respective mixture and in case of (iii) at least one mixture is formed, enriched in anthocyans of delphinidine, cyanidine, petunidine, peonidine and malvidine type in its glyosidic form and additionally gallic acid and sinapic acid - calculated on the total mass of dry matter - and wherein the ratio in the mixture of in anthocyans of delphinidine, cyanidine, petunidine, peonidine and malvidine type in its glyosidic form on one hand and gallic acid and sinapic acid on the other ranges from (3-8):(1.8-6):(0.5-5):1:(0.5-5.5):(0.005-1):(0.00005-0.01).

8. The process of Claim 7, wherein in step (a) in case of (i) the juice or respectively in case of (ii) the extract or in case of (iii) the total of juice and extract contains anthocyans of delphinidine, cyanidine, petunidine, peonidine and malvidine type in its glyosidic form and additionally gallic acid and sinapic acid, wherein the ratio of the total masses of anthocyans of delphinidine, cyanidine, petunidine, peonidine and malvidine type in its glyosidic form and additionally gallic acid and sinapic acid in (i) said juice, (ii) said extract or respectively (iii) said total of juice and extract ranges from (3-8):(1.8-6):(0.5-5):1(0.5-5.5):<0.06:<0.001.

9. The process of any of the preceding claims, wherein in step (b) separation in case of (i) of the juice or respectively (ii) the extract or (iii) the total of juice and extract encompasses:

(b) (1) bringing in contact (i) said juice or respectively (ii) said extract or (iii) said total of juice and extract with an adsorbent, capable of selectively binding anthocyans of delphinidine, cyanidine, petunidine, peonidine and malvidine type in its glyosidic form and additionally also said hydroxy benzoic acids and hydroxy cinnamic acids when compared to sugars,
(b) (2) desorbing at least said anthocyans of delphinidine, cyanidine, petunidine, peonidine and malvidine type in its glyosidic form bounded to said adsorbent.

10. The process of claim 9, wherein in step (b) (2) also hydroxy benzoic acids and hydroxy cinnamic acids are desorbed.

11. The process of claim 9 or 10, wherein in step (b) (2) desorbing takes places, in that said adsorbent is brought into contact with one, two or more solvents, which are preferably selected from the group consisting of water, ethanol or their mixtures.

12. The process of any of the preceding claims, comprising the following steps:

(a) making or providing (ii) a juice as well as an extract,
(b) separating (ii) said juice and as well said extract, wherein a mixture is obtained both from said juice and from said extract, comprising delphinidine, cyanidine, petunidine, peonidine and malvidine, and
wherein at least one mixture is formed, enriched in delphinidine, cyanidine, petunidine, peonidine and malvidine and depleted of sugars - calculated on the respective total mass of dry matter-
(c) optionally drying of the mixtures resulting from step (b), preferably up to a dry matter amount of individually at least 80 wt.-%, more preferably up to a dry matter amount of individually at least 90 wt.-%,

(d) combining the mixture of the juice according to step (b) or step (c) with the mixture obtained from the extract,

to obtain a joint mixture wherein the ratio of the total masses of anthocyans of delphinidine, cyanidine, petunidine, peonidine and malvidine type in its glyosidic form ranges from (3-8):(1.8:6):(0.5-5):1:(0.5-5.5).

13. Composition comprising anthocyans of delphinidine, cyanidine, petunidine, peonidine and malvidine type in its glyosidic form and additionally gallic acid and sinapic acid, wherein the ratio of anthocyans of delphinidine, cyanidine, petunidine, peonidine and malvidine type in its glyosidic form on one hand and gallic acid and sinapic acid on the other ranges in the mixture from (3-8):(1.8-6):(0.5-5):1:(0.5-5.5):(0.005-1):(0.00005-0.01).

14. The composition of claim 14, wherein the total amount of mono and disaccharides is less than 2 wt.-%, preferably less than 1.5 wt.-% - calculated on the dry matter of the composition.

15. The composition of claim 13 or 14, obtainable according to a process of any of the claims 1 to 12.

## Revendications

1. Procédé de formation d'une composition comprenant les anthocyanes delphinidine, cyanidine, pétunidine, péonidine et malvidine sous forme reliée à un glycoside, le rapport entre les masses totales des anthocyanes reliés à un glycoside delphinidine, cyanidine, pétunidine, péonidine et malvidine dans la composition se situant dans la plage de (3-8):(1,8-6):(0,5-5):1:(0,5-5,5),
comprenant les étapes suivantes :

   (a) la fabrication ou la préparation

      (i) d'un jus ou
      (ii) d'un extrait ou
      (iii) aussi bien d'un jus que d'un extrait,

   (i) le jus ou (ii) l'extrait ou (iii) le jus et l'extrait étant fabriqués à partir de fruits de *Vaccinium myrtillus* L. et
   (i) le jus ou (ii) l'extrait ou (iii) l'ensemble du jus et de l'extrait comprenant tous les anthocyanes delphinidine, cyanidine, pétunidine, péonidine et malvidine sous forme reliée à un glycoside, ainsi que des sucres,
   (b) la séparation (i) du jus ou (ii) de l'extrait ou (iii) aussi bien du jus que de l'extrait, de manière à former dans les cas (i) et (ii) un mélange respectif qui, par rapport à la masse totale respective de substance sèche, est enrichi en les anthocyanes reliés à un glycoside delphinidine, cyanidine, pétunidine, péonidine et malvidine et à former dans le cas (iii) deux mélanges parmi lesquels au moins un, par rapport à la masse totale respective de substance sèche, est enrichi en les anthocyanes reliés à un glycoside delphinidine, cyanidine, pétunidine, péonidine et malvidine,

   le rapport entre les masses totales des anthocyanes reliés à un glycoside delphinidine, cyanidine, pétunidine, péonidine et malvidine dans les cas (i) et (ii) dans le mélange respectif et dans le cas (iii) dans l'ensemble des deux mélanges se situant dans la plage de (3-8):(1,8-6):(0,5-5):1:(0,5-5,5).

2. Procédé selon la revendication 1, dans lequel, lors de la séparation (i) du jus ou (ii) de l'extrait ou (iii) aussi bien du jus que de l'extrait à l'étape (b), dans les cas (i) et (ii), un mélange respectif est formé qui, par rapport à la masse totale respective de substance sèche, est appauvri en sucres, et dans le cas (iii), deux mélanges sont formés, parmi lesquels au moins un, par rapport à la masse totale respective de substance sèche, est appauvri en sucres.

3. Procédé selon la revendication 1 ou 2, dans lequel, à l'étape (a), dans le cas (i) le jus ou dans le cas (ii) l'extrait ou dans le cas (iii) l'ensemble du jus et de l'extrait contient tous les anthocyanes delphinidine, cyanidine, pétunidine, péonidine et malvidine sous forme reliée à un glycoside, le rapport entre les masses totales des anthocyanes reliés à un glycoside delphinidine, cyanidine, pétunidine, péonidine et malvidine dans (i) le jus, (ii) l'extrait ou (iii) l'ensemble du jus et de l'extrait se situe dans la plage de (3-8):(1,8-6):(0,5-5):1:(0,5-5,5).

4. Procédé selon l'une quelconque des revendications 1 à 3, dans lequel, à l'étape (a), dans le cas (i) le jus ou dans le cas (ii) l'extrait ou dans le cas (iii) l'ensemble du jus et de l'extrait contient tous les anthocyanes delphinidine, cyanidine, pétunidine, péonidine et malvidine sous forme reliée à un glycoside, ainsi que des sucres, et contient en outre

- un ou plusieurs acides hydroxybenzoïques, ou
- un ou plusieurs acides hydroxycinnamiques, ou
- un ou plusieurs acides hydroxybenzoïques et un ou plusieurs acides hydroxycinnamiques,

à l'étape (b), dans les cas (i) et (ii), un mélange respectif et dans le cas (iii) au moins un mélange étant formé qui, par rapport à la masse totale respective de substance sèche, est enrichi en le ou les acides hydroxybenzoïques ou le ou les acides hydroxycinnamiques ou le ou les acides hydroxybenzoïques et le ou les acides hydroxycinnamiques.

5. Procédé selon la revendication 4, dans lequel le ou un des acides hydroxybenzoïques est l'acide gallique, et/ou le ou les acides hydroxycinnamiques sont choisis dans le groupe constitué par l'acide sinapique, l'acide férulique, l'acide caféique, l'acide ellagique, l'acide cumarique et/ou l'acide chlorogénique.

6. Procédé selon l'une quelconque des revendications précédentes, dans lequel, à l'étape (a), dans le cas (i) le jus ou dans le cas (ii) l'extrait ou dans le cas (iii) l'ensemble du jus et de l'extrait, contient tous les anthocyanes delphinidine, cyanidine, pétunidine, péonidine et malvidine sous forme reliée à un glycoside, ainsi que des sucres et contient en outre de l'acide gallique et de l'acide sinapique, à l'étape (b), dans les cas (i) et (ii) un mélange respectif et dans le cas (iii) au moins un mélange étant formé qui, par rapport à la masse totale respective de substance sèche, est enrichi en acide gallique et en acide sinapique.

7. Procédé selon la revendication 6, dans lequel à l'étape (b), dans les cas (i) et (ii) un mélange respectif et dans le cas (iii) au moins un mélange est formé qui, par rapport à la masse totale respective de substance sèche, est enrichi en les anthocyanes reliés à un glycoside delphinidine, cyanidine, pétunidine, péonidine et malvidine, ainsi qu'en acide gallique et en acide sinapique, le rapport entre les masses totales des anthocyanes reliés à un glycoside delphinidine, cyanidine, pétunidine, péonidine et malvidine, ainsi que de l'acide gallique et de l'acide sinapique dans le mélange se situant dans la plage de (3-8):(1,8-6):(0,5-5):1:(0,5-5,5):(0,005-1):(0,00005-0,01).

8. Procédé selon la revendication 7, dans lequel, à l'étape (a), dans le cas (i) le jus ou dans le cas (ii) l'extrait ou dans le cas (iii) l'ensemble du jus et de l'extrait contient tous les anthocyanes delphinidine, cyanidine, pétunidine, péonidine et malvidine sous forme reliée à un glycoside, ainsi que de l'acide gallique et de l'acide sinapique, le rapport entre les masses totales des anthocyanes reliés à un glycoside delphinidine, cyanidine, pétunidine, péonidine et malvidine, ainsi que de l'acide gallique et de l'acide sinapique dans (i) le jus, (ii) l'extrait ou (iii) l'ensemble du jus et de l'extrait se situant dans la plage de (3-8):(1,8-6):(0,5-5):1:(0,5-5,5):< 0,06:< 0,001.

9. Procédé selon l'une quelconque des revendications précédentes, dans lequel, à l'étape (b), la séparation dans le cas (i) du jus ou dans le cas (ii) de l'extrait ou dans le cas (iii) du jus et de l'extrait comprend les étapes de séparation suivantes :

(b)(1) la mise en contact de (i) le jus ou (ii) l'extrait ou (iii) respectivement le jus et l'extrait avec un adsorbant, auquel les anthocyanes reliés à un glycoside delphinidine, cyanidine, pétunidine, péonidine et malvidine, ainsi que les acides hydroxybenzoïques et les acides hydroxycinnamiques se lient sélectivement en comparaison des sucres,
(b)(2) la désorption d'au moins les anthocyanes reliés à un glycoside delphinidine, cyanidine, pétunidine, péonidine et malvidine reliés à l'adsorbant.

10. Procédé selon la revendication 9, dans lequel, à l'étape (b)(2), les acides hydroxybenzoïques et les acides hydroxycinnamiques sont également désorbés.

11. Procédé selon la revendication 9 ou 10, dans lequel, à l'étape (b)(2), la désorption a lieu par mise en contact de l'adsorbant avec un, deux solvants ou plus, qui sont de préférence choisis dans le groupe constitué par l'eau, l'éthanol et leurs mélanges.

12. Procédé selon l'une quelconque des revendications précédentes, comprenant les étapes suivantes :

(a) la fabrication ou la préparation de (iii) aussi bien un jus qu'un extrait,
(b) la séparation (iii) aussi bien du jus que de l'extrait,
un mélange résultant du jus et un mélange résultant de l'extrait, qui comprennent chacun de la delphinidine, de la cyanidine, de la pétunidine, de la péonidine et de la malvidine,

au moins un mélange étant formé, qui, par rapport à la masse totale respective de substance sèche, est enrichi en les anthocyanes delphinidine, cyanidine, pétunidine, péonidine et malvidine, et appauvri en sucres,

(c) éventuellement le séchage des mélanges présents après l'étape (b), de préférence à chaque fois jusqu'à une teneur en substance sèche d'au moins 80 % en poids, de préférence jusqu'à une teneur en substance sèche d'au moins 90 % en poids,

(d) la réunion du mélange résultant du jus selon l'étape (b) ou selon l'étape (c) avec le mélange résultant de l'extrait,

de sorte que, dans le mélange réuni, le rapport entre les masses totales des anthocyanes reliés à un glycoside delphinidine, cyanidine, pétunidine, péonidine et malvidine soit de (3-8):(1,8-6):(0,5-5):1:(0,5-5,5).

13. Composition comprenant les anthocyanes reliés à un glycoside delphinidine, cyanidine, pétunidine, péonidine et malvidine, ainsi que de l'acide gallique et de l'acide sinapique, le rapport entre les masses totales des anthocyanes reliés à un glycoside delphinidine, cyanidine, pétunidine, péonidine et malvidine, ainsi que de l'acide gallique et de l'acide sinapique dans le mélange se situant dans la plage de (3-8):(1,8-6):(0,5-5):1:(0,5-5,5):(0,005-1):(0,00005:0,01).

14. Composition selon la revendication 13, dans laquelle la proportion totale de mono- et disaccharides est inférieure à 2 % en poids, de préférence inférieure à 1,5 % en poids, par rapport à la substance sèche de la composition.

15. Composition selon les revendications 13 ou 14, pouvant être fabriquée au moyen d'un procédé selon l'une quelconque des revendications 1 à 12.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

*   US 2007269536 A1

**In der Beschreibung aufgeführte Nicht-Patentliteratur**

*   Phenolic Content and Radical Scavenging Capacity of Berries and related Jams from certificated area in Serbia. **KATARINA AA AVIKIN.** Plant Foods for Human Nutrition. Kluwer Academic Publishers, DO, 26. Mai 2009, vol. 64, 212-217

*   **REINHARD MATISSEK ; GABRIELE STEINER ; FRANK-M.** Schnepel: Lebensmittelanalytik: Grundzüge, Methoden, Anwendungen. Springer-Verlag, 1992, 118ff